# EUROPEAN PATENT APPLICATION

(11) **EP 2 332 608 A2**
(43) Date of publication of application: **15.06.2011**
(21) Application number: 10190400.1
(22) Date of filing: 08.11.2010
(51) Int. Cl.: A61M 25/09

(54) **Guidewire**

(30) Priority: 10.12.2009 JP 2009280881
(71) Applicant: ASAHI INTECC CO., LTD., Nagoya-shi, Aichi 463-0024 (JP)
(72) Inventor: Shimogami, Manabu, Nagoya-shi Aichi 463-0024 (JP); Kitagawa, Masanori, Nagoya-shi Aichi 463-0024 (JP)
(74) Representative: Stöckeler, Ferdinand

(57) **Abstract**

Provided is a guidewire (10) effectively usable for a retrograde approach. A guidewire has a body portion (20), and a first tip portion (30) and a second tip portion (60) provided at ends of the body portion. Flexural stiffnesses of the first tip portion (30) and the second tip portion (60) increase in a continuous or stepwise manner from the respective tips of the first and second tip portions toward the body portion. The flexural stiffness of the first tip portion is not smaller in value than the flexural stiffness of the second tip portion in first distance ranges from the respective tips of the first and second tip portions toward the body portion. The flexural stiffness of the first tip portion is smaller in value than the flexural stiffness of the second tip portion in second distance ranges beyond the respective first distance ranges of the first and second tip portions toward the body portion.

## Description

### Cross Reference to Related Applications

This application is based on Japanese Patent Application No. 2009-280881 filed with the Japan Patent Office on December 10, 2009, the entire content of which is hereby incorporated by reference.

### Technical Field

The present invention relates to a medical guidewire.

### Background Art

There have hitherto been proposed a variety of medical guidewires for guiding, e.g., a catheter which is used by being inserted into tubular organs such as blood vessels, digestive tracts, and ureters, and intracorporeal tissues (e.g., see JP 2003-052831A and U. S. Patent No. 5363847).

Further, there has recently been proposed an operation called a retrograde approach as a cardiac catheterization treatment method performed with use of a guidewire (e.g., see US 2007/0208368 A1). This approach is not an operation for directly approaching a stenosis as a target treatment area. More specifically, this approach is an operation for approaching the target stenosis from a direction opposite a conventional direction through a special blood vessel such as a thin blood vessel called a collateral channel.

For example, when the stenosis as the target treatment area is present in a right coronary artery, a normal method for approaching the stenosis from the right coronary artery ostium is called an antegrade approach, whereas a retrograde approach is an operation for approaching the stenosis in the right coronary artery from a left coronary artery ostium and passing through a collateral channel, to reach the stenosis so as to perform a treatment.

The retrograde approach is suitable for treatment of a stenosis in a relatively severe state which is difficult to treat by the conventional antegrade approach.

### Summary of Invention

The retrograde approach is an operation with a concept different from that of the antegrade approach that has hitherto been performed. Hence, as illustrated in US 2007/0208368 A1, attempts have been made to use a variety of new medical devices, and such devices have been developed.

The present invention was made in view of the foregoing circumstances. An object of the present invention is to provide a guidewire effectively usable for the retrograde approach.

In the present invention, the above object is achieved by the following features.

A guidewire in accordance with the invention is a guidewire having a first tip portion and a second tip portion at ends of a body portion, wherein flexural stiffnesses of the first tip portion and the second tip portion increase in a continuous or stepwise manner from the respective tips of the first tip portion and the second tip portion toward the body portion, the flexural stiffness of the first tip portion is not smaller in value than the flexural stiffness of the second tip portion in first distance ranges from the respective tips of the first tip portion and the second tip portion toward the body portion, and the flexural stiffness of the first tip portion is smaller in value than the flexural stiffness of the second tip portion in second distance ranges beyond the respective first distance ranges of the first tip portion and the second tip portion toward the body portion.

### Brief Description of Drawings

The foregoing and other objects, features, aspects and advantages of the invention will become more apparent from the following detailed description when taken in conjunction with the accompanying drawings.
Fig. 1

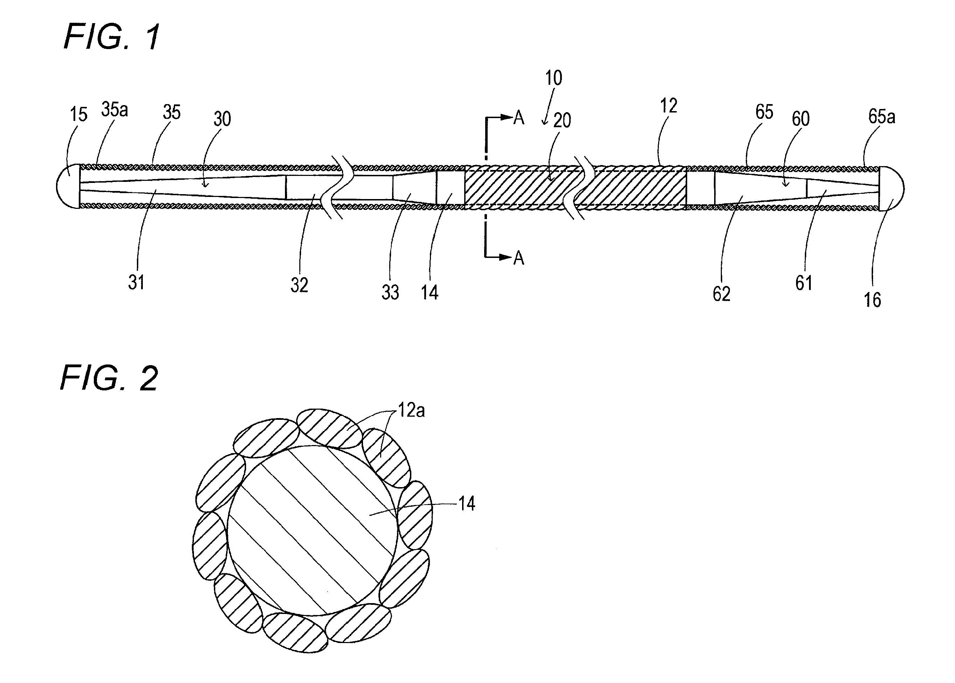

   Fig. 1 illustrates an overall view of a guidewire of an embodiment of the present invention.
Fig. 2
   Fig. 2 illustrates a cross-sectional view taken along line A-A of Fig. 1.
Fig. 3

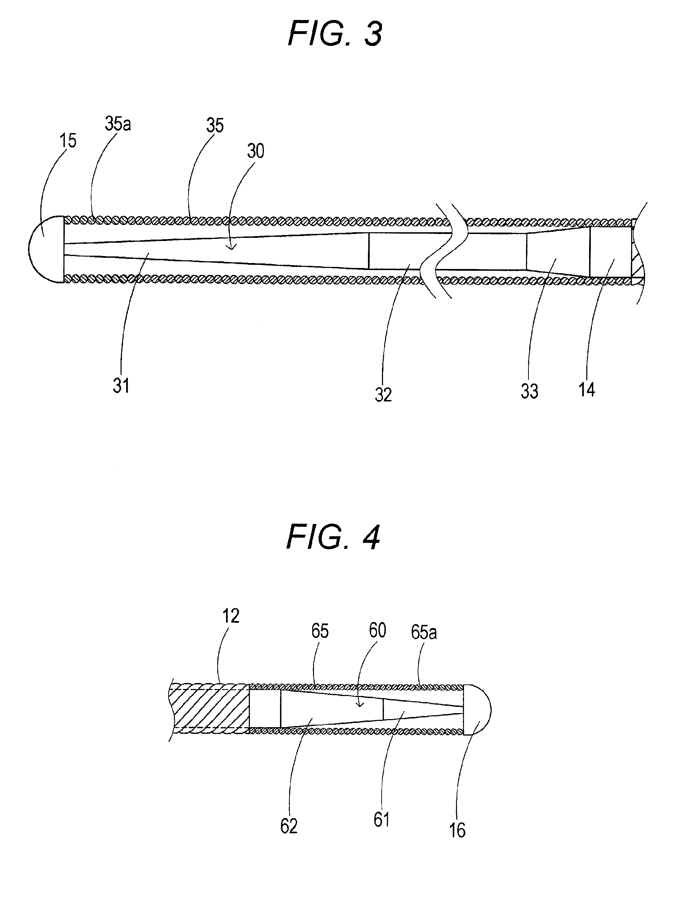

   Fig. 3 illustrates a first tip portion of the guidewire of the embodiment.
Fig. 4
   Fig. 4 illustrates a second tip portion of the guidewire of the embodiment.
Fig. 5

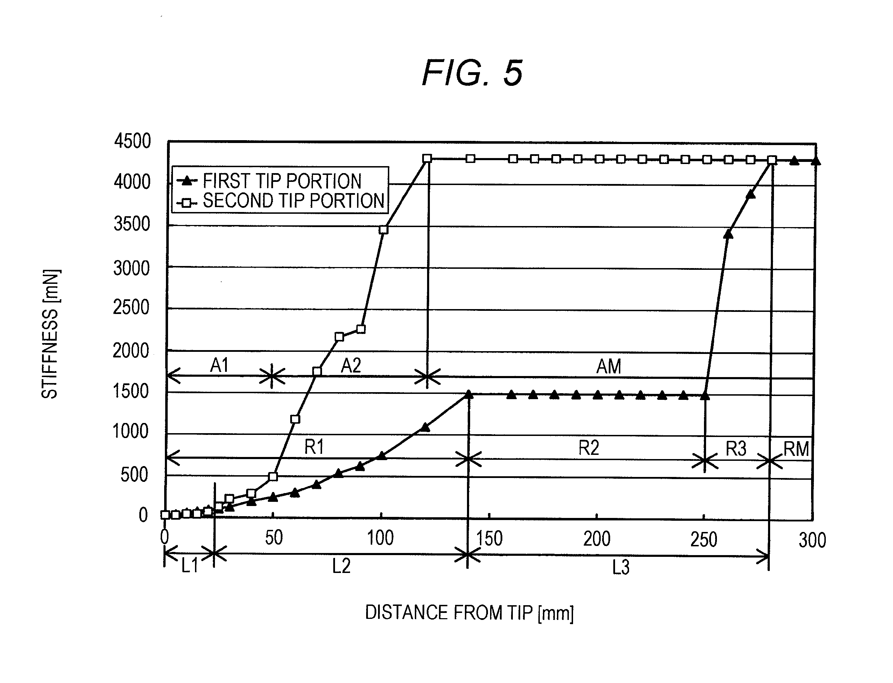

   Fig. 5 illustrates a graph showing flexural stiffness of the guidewire of the embodiment.
Fig. 6

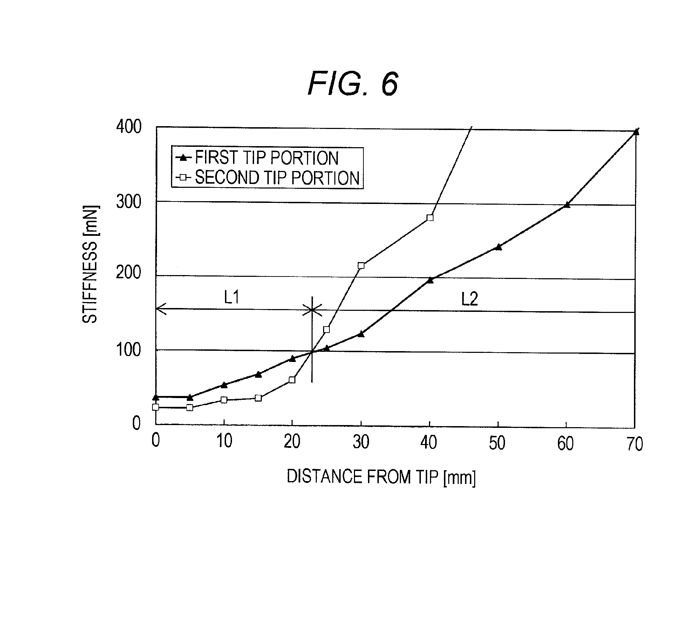

   Fig. 6 illustrates a partially enlarged view of Fig. 5.
Fig. 7

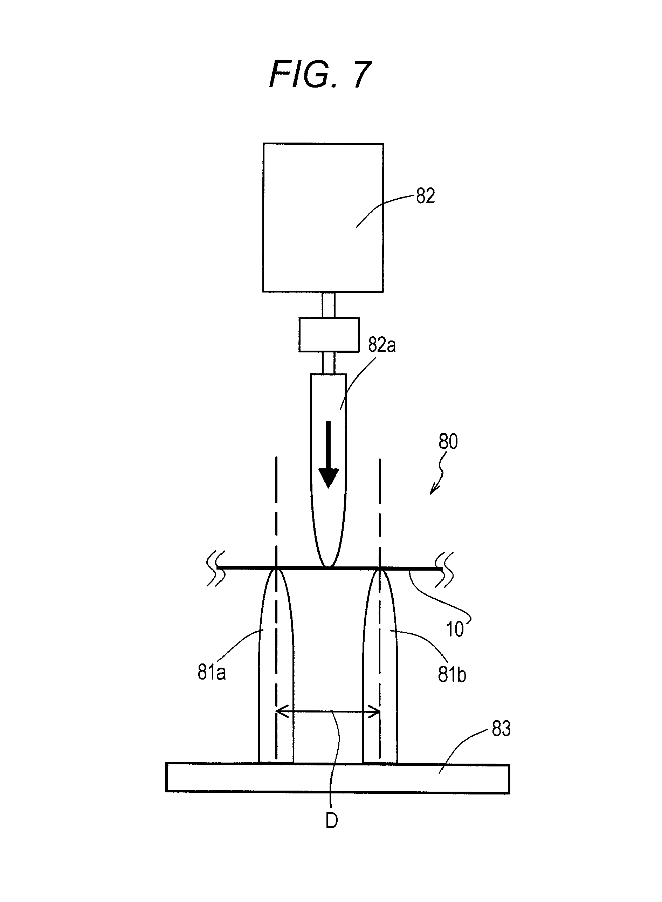

   Fig. 7 illustrates a measurement device for the data of Fig. 5.
Fig. 8

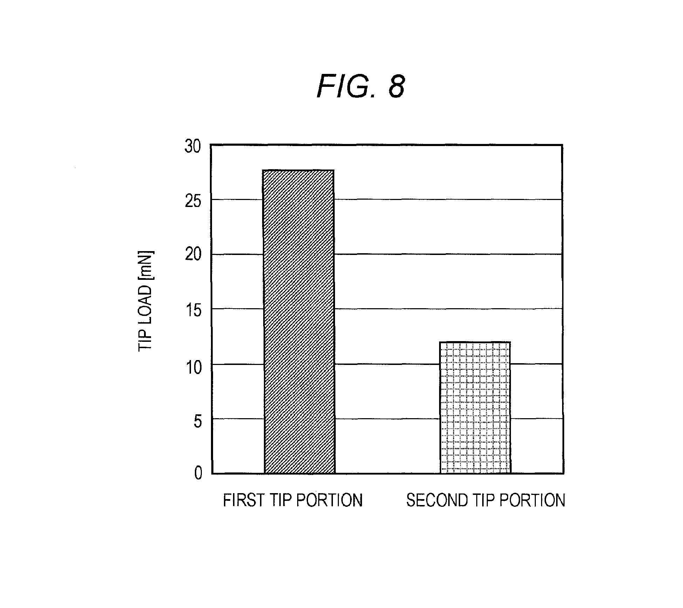

   Fig. 8 illustrates a graph showing a tip load of the guidewire of the embodiment.
Fig. 9

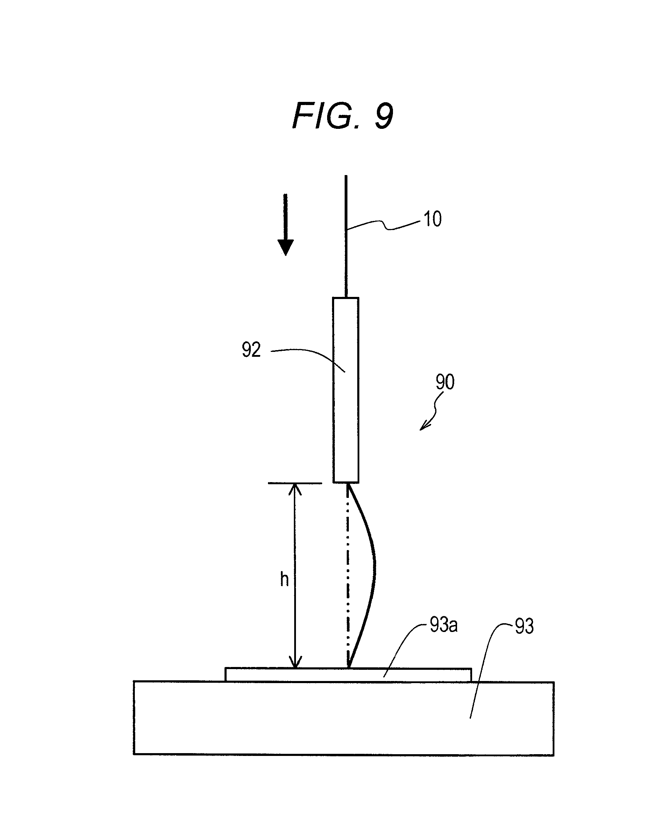

   Fig. 9 illustrates a measurement device for the data of Fig. 8.
Fig. 10

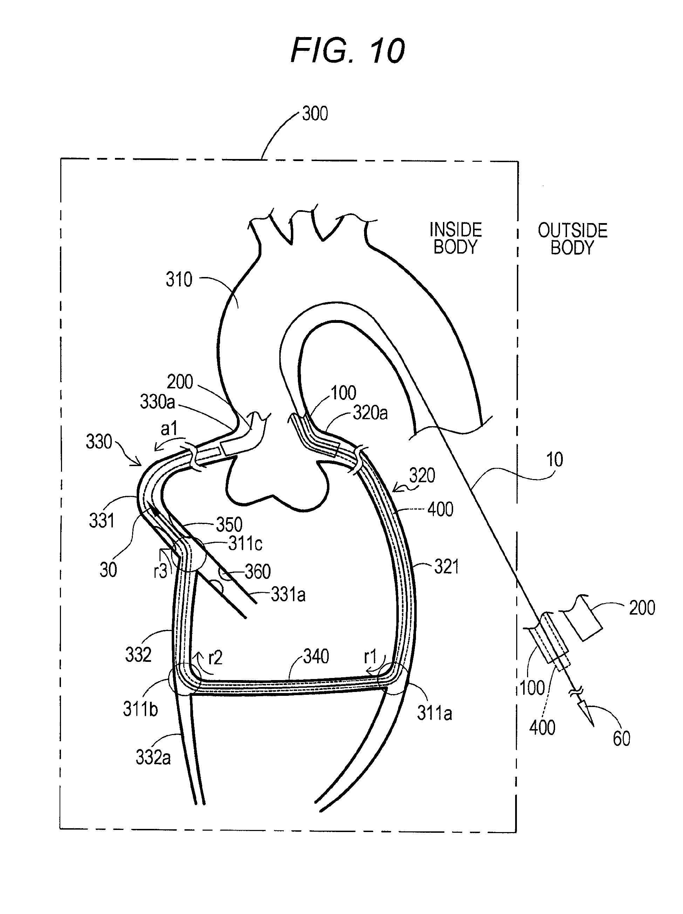

   Fig. 10 illustrates an explanatory drawing of a function of the guidewire of the embodiment.
Fig. 11

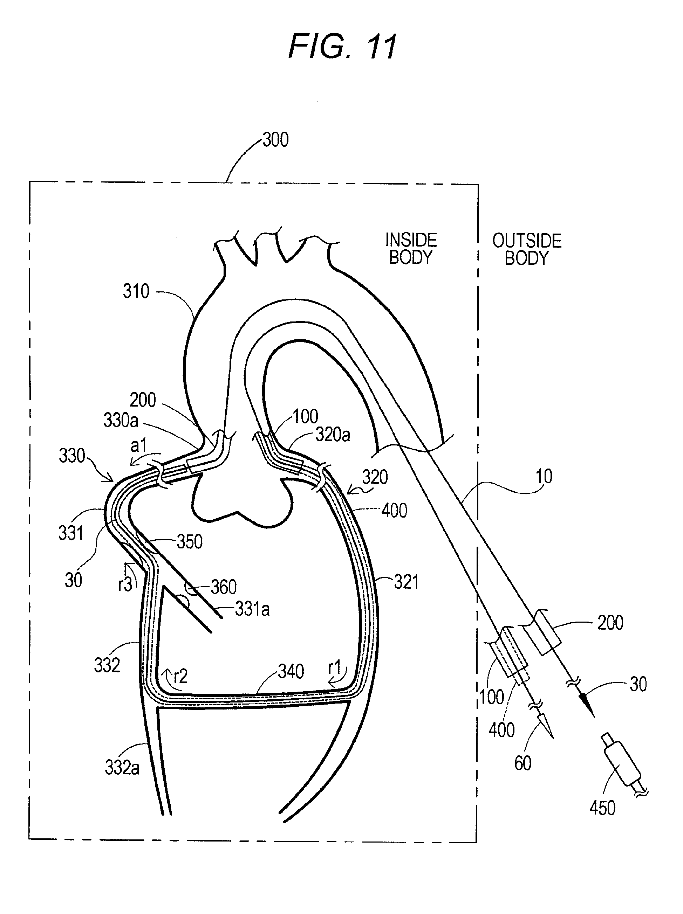

   Fig. 11 illustrates an explanatory drawing, following Fig. 10, of the function of the guidewire of the embodiment.
Fig. 12

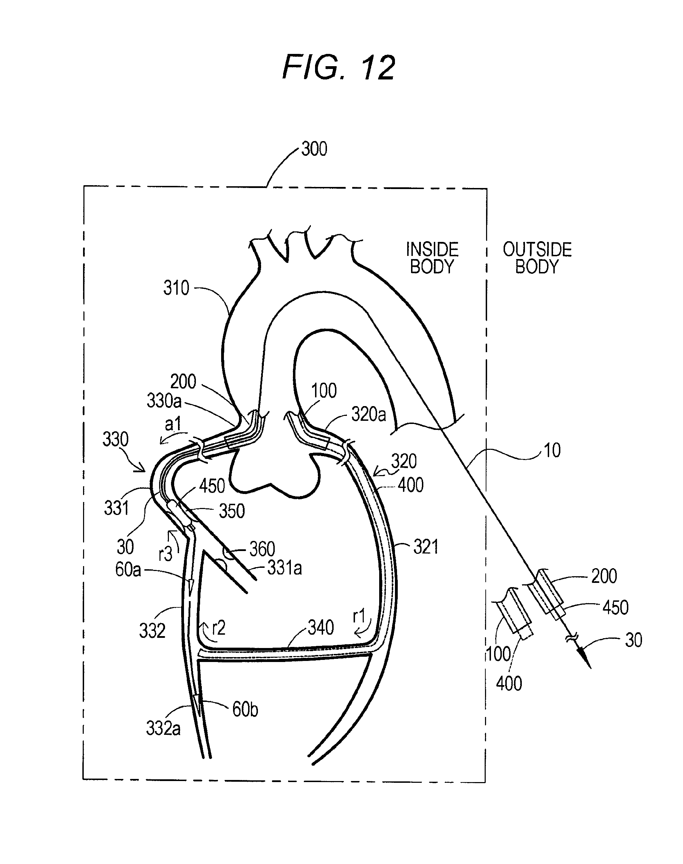

   Fig. 12 illustrates an explanatory drawing, following Fig. 11, of the function of the guidewire of the embodiment.
Fig. 13

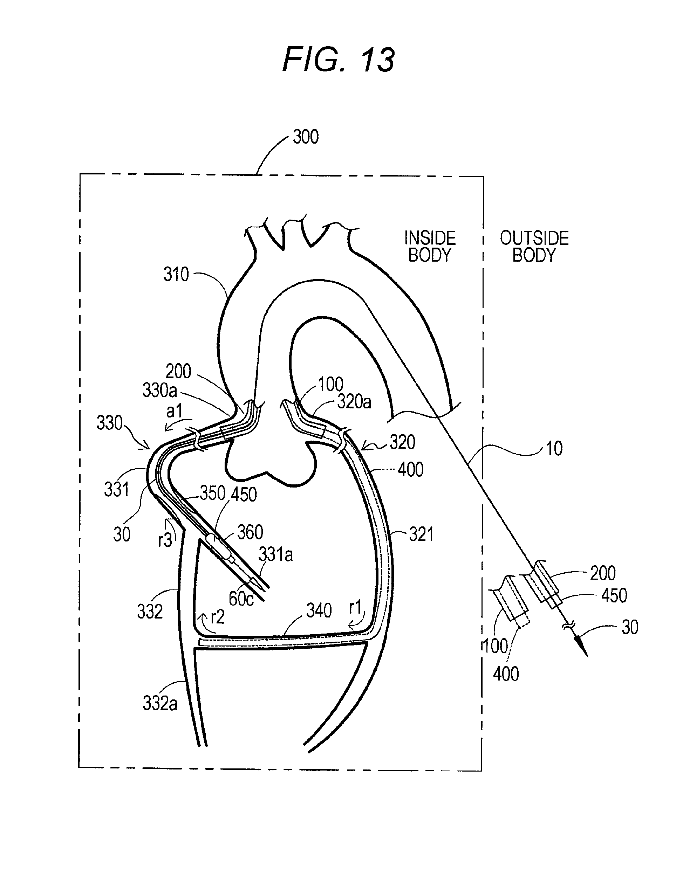

   Fig. 13 is an explanatory drawing, following Fig. 12, of the function of the guidewire of the embodiment.
Fig. 14

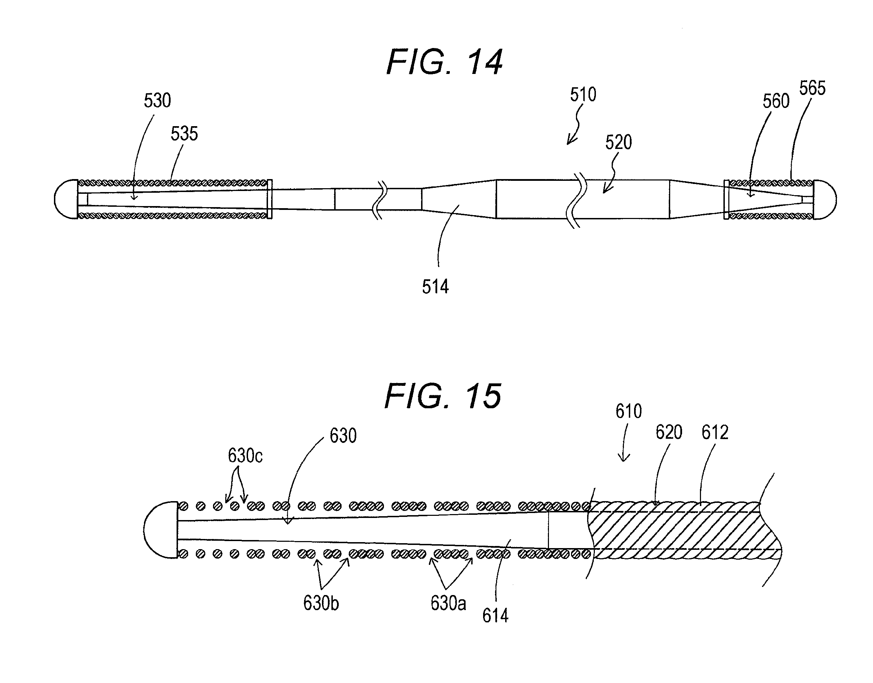

   Fig. 14 illustrates a second embodiment.
Fig. 15
   Fig. 15 illustrates a third embodiment.

### Description of Embodiments

<1> A guidewire in accordance with a first aspect of the invention is a guidewire having a first tip portion and a second tip portion at ends of a body portion, wherein flexural stiffnesses of the first tip portion and the second tip portion increase in a continuous or stepwise manner from the respective tips of the first tip portion and the second tip portion toward the body portion, the flexural stiffness of the first tip portion is not smaller in value than the flexural stiffness of the second tip portion in first distance ranges from the respective tips of the first tip portion and the second tip portion toward the body portion, and the flexural stiffness of the first tip portion is smaller in value than the flexural stiffness of the second tip portion in second distance ranges beyond the respective first distance ranges of the first tip portion and the second tip portion toward the body portion.

<2> A guidewire in accordance with a second aspect of the invention is the guidewire of the first aspect, wherein the first tip portion is larger in tip load than the second tip portion.

<3> A guidewire in accordance with a third aspect of the invention is the guidewire of the second aspect, wherein the tip load of the first tip portion is not smaller than 24 mN.

<4> A guidewire in accordance with a fourth aspect of the invention is the guidewire of the first aspect, wherein the first tip portion is larger in axial length than the second tip portion.

<5> A guidewire in accordance with a fifth aspect of the invention is the guidewire of the fourth aspect, wherein the axial length of the first tip portion is not shorter than 150 mm.

<6> A guidewire in accordance with a sixth aspect of the invention is the guidewire of the first aspect, wherein the guidewire has such an overall length as to allow the first tip portion to be inserted into a body, pass through an inside of the heart, be turned back and exposed to an outside of the body in a state where the second tip portion is exposed to the outside of the body.

<7> A guidewire in accordance with a seventh aspect of the invention is the guidewire of the first aspect, wherein the body portion is configured with a core wire being inserted through an inside of a stranded coil made up of a plurality of strands.

<1> A guidewire in accordance with the first aspect of the invention has, at the ends of the body portion, the first tip portion preferable for the retrograde approach and the second tip portion preferable for the antegrade approach. The flexural stiffness of the first tip portion is set so as to moderately increase in the first distance range and the second distance range. Therefore, the first tip portion is capable of entering into a bent or tortuous blood vessel, and is thus suitable for treatment of a stenosis by the retrograde approach. Meanwhile, the flexural stiffness of the first tip portion in the first distance range in the vicinity of the tip is set high as compared with the flexural stiffness of the second tip portion in the corresponding portion. This can facilitate insertion of a medical catheter, such as a balloon catheter, at the time of the first tip portion being exposed to the outside of the body.

The flexural stiffness of the second tip portion is set relatively low and flexible in the first distance range. Thereby, the second tip portion is suitable for proceeding to a blood vessel having no stenosis, or a stenosis in a relatively nonsevere state, by operating the guidewire by the antegrade approach. The flexural stiffness of a portion transiting from the first distance range to the second distance range is preferably relatively high for supporting the medical catheter. Hence the flexural stiffness of the second tip portion in the second distance range is set high as compared with the flexural stiffness of the first tip portion in the corresponding portion.

With such a configuration, the first tip portion used for the retrograde approach and the second tip portion used for the antegrade approach are capable of being used continuously. It is thereby possible to perform treatment of a stenosis in a severe state, which has been difficult by the conventional operation, and also successively treat a stenosis by the antegrade approach other than the stenosis treated by the retrograde approach.

<2> In the second aspect of the invention, the tip load of the first tip portion is set larger than the tip load of the second tip portion. Thereby, when the first tip portion is exposed to the outside of the body by the retrograde approach, a medical catheter such as a balloon catheter is easily inserted into the first tip portion from the tip thereof. Further, with the tip of the second tip portion being flexible, even when the tip contacts with the inner wall or the like of a blood vessel at the time of the antegrade approach, damaging the inner wall of the blood vessel or the like is prevented as much as possible.

<3> In the third aspect of the invention, the tip load of the first tip portion is set to not smaller than 24 mN. This remarkably facilitates insertion of a medical catheter such as a balloon catheter from the tip of the first tip portion.

<4> In the fourth aspect of the invention, the axial length of the first tip portion is set larger than the axial length of the second tip portion. Therefore, also at the time of the first tip portion passing from a first coronary artery through a blood vessel called "collateral channel" which is thin and bent at a relatively acute angle and proceeding to a second coronary artery, the first tip portion is capable of flexibly following and proceeding along the blood vessel. Further, even in the case of the guidewire proceeding so as to cross inside the heart, a load on the heart is prevented as much as possible by the first tip portion being long and flexible.

<5> In the fifth aspect of the invention, the axial length of the first tip portion is set not less than 150 mm. Thereby, the first tip portion is long enough to flexibly follow and proceed along the blood vessel at the time of entering a collateral channel from a first coronary artery and reaching a second coronary artery. As thus described, load on the heart is prevented as much as possible by the first tip portion being long and flexible.

<6> In the sixth aspect of the invention, the guidewire has such an overall length as to allow the first tip portion to be inserted into a body, pass through the inside of the heart, be turned back and exposed to the outside of the body in a state where the second tip portion is exposed to the outside of the body. This facilitates continuous use of the first tip portion used for the retrograde approach and the second tip portion used for the antegrade approach.

<7> In the seventh aspect of the invention, the body portion is configured with a core wire being inserted through the inside of a stranded coil made up of a plurality of strands. For this reason, forming a desired outer diameter from the stranded coil and also using a core wire that fits therewith enables subtle adjustment of the outer diameter and the stiffness of the guidewire. Further, using the stranded coil enables achievement of a flexible guidewire with high torque transmissibility.

A guidewire of the present embodiment is described with reference to Figs. 1 to 4.

A guidewire 10 is used for cardiac treatment. As described later, the guidewire 10 needs to be long enough to enter into a body from the outside thereof through, e.g., a wrist region or a femoral region, pass through the heart, and be again exposed to the outside of the body. Therefore, the guidewire 10 has a length of not shorter than about 2600 mm, and a length of about 3300 mm in the case of the present embodiment. Considering that a normal guidewire has a length on the order of 1800 mm, the guidewire 10 has a length at least more than 1.4 times longer than that of the normal one.

In Fig. 1, the guidewire 10 has a first tip portion 30, a body portion 20, and a second tip portion 60 sequentially from the left.

The first tip portion 30 is used for a retrograde approach. On the other hand, the second tip portion 60 is used for the antegrade approach after the retrograde approach. The axial length of the first tip portion 30 is set not shorter than 150 mm, and about 280 mm in the present embodiment. The axial length of the second tip portion 60 is set to a range on the order of 40 to 150 mm, and about 120 mm in the present embodiment. The body portion 20 makes up a portion other than the first tip portion 30 and the second tip portion 60, and its length is about 2900 mm in the present embodiment.

The guidewire 10 has a core wire 14. In the body portion 20, the core wire 14 has a cylindrical shape. A portion with a substantial length of the core wire 14 other than the tip portions is inserted through a stranded coil 12. The stranded coil 12 is manufactured by stranding a plurality of metal strands 12a in a hollow shape, and then removing residual stress generated at the time of stranding by a known thermal treatment. The outer diameter of the stranded coil 12 is preferably on the order of 0.25 to 0.45 mm but is not limited thereto. The outer diameter of this stranded coil 12 is about 0.35 mm in the present embodiment.

The size of this outer diameter is set in light of the inner diameter of a guidewire lumen for the medical catheter, such as a balloon catheter, into which the guidewire 10 is inserted.

Ten strands 12a are used for the stranded coil 12. The outer diameter of a strand 12a is about 0.06 mm. The number and the outer diameters of the strands 12a are suitably decided in light of a required outer diameter and stiffness of the stranded coil 12, and are not restricted to the above values.

Although the material for the strands 12a is not particularly restricted, stainless steel is used in the case of the present embodiment. As a material other than this, a super elastic alloy such as an Ni-Ti alloy is used. Further, strands made from different materials may be combined.

It is to be noted that in Fig. 2, cross sections of the strands 12a are drawn to be elliptical. This is because in a cross section orthogonal to the axis of the core wire 14, the cross section of the strands 12a spirally stranded appears elliptical. In a cross section orthogonal to the axis of a strand 12a, the cross section of the strand 12a is substantially circular.

The outer diameter of the core wire 14 in the body portion 20 is about 0.22 mm in the case of the present embodiment.

Although the material for the core wire 14 is not particularly restricted, in the case of the present embodiment, stainless steel (SUS304 according to the JIS standard) is used. As the material other than this, a super elastic alloy such as an Ni-Ti alloy and a piano wire are used.

In the first tip portion 30 and the second tip portion 60, the core wire 14 is formed with a taper. Therefore, the diameter of the core wire 14 decreases toward the tips of the respective tip portions 30 and 60.

In the first tip portion 30 illustrated in Fig. 3, the core wire 14 is provided with a first taper part 31, a first small diameter part 32, and a second taper part 33 sequentially from the tip toward the body portion 20. In the present embodiment, the axial length of the first taper part 31 is about 140 mm, the axial length of the first small diameter part 32 is about 110 mm, and the axial length of the second taper part 33 is about 30 mm. Further, the outer diameter of the tip of the first taper part 31 is about 0.07 mm, and the outer diameter of the first small diameter part 32 is about 0.18 mm.

In the first tip portion 30, the core wire 14 is inserted through a first coil 35 formed by spirally winding a single strand. The end portion on the tip side of the first coil 35 is joined to the tip of the first tip portion 30 by brazing. This brazed part forms a tip plug 15 in a substantially semispherical shape. The end portion on the body portion 20 side of the first coil 35 extends to the body portion 20 and is joined to the end portion on the first tip portion 30 side of the stranded coil 12 by brazing. As a material for a principal portion of the first coil 35, stainless steel is used in the case of the present embodiment. Meanwhile, a portion with a length on the order of 30 mm at the tip side portion of the first coil 35 is a marker part 35a formed of radiopaque metal such as platinum.

In the second tip portion 60 illustrated in Fig. 4, the core wire 14 is provided with a third taper part 61 and a fourth taper part 62 sequentially from the tip toward the body portion 20. The third taper part 61 and the fourth taper part 62 are tapers with different inclination angles. In the present embodiment, the axial length of the third taper part 61 is about 50 mm, and the axial length of the fourth taper part 62 is about 70 mm. Further, the outer diameter of the tip of the third taper part 61 is about 0.06 mm.

In the second tip portion 60, the core wire 14 is inserted through a second coil 65 formed by spirally winding a single strand. The end portion on the tip side of the second coil 65 is joined to the tip of the second tip portion 60 by brazing. This brazed part forms a tip plug 16 in a substantially semispherical shape. The end portion on the body portion 20 side of the second coil 65 extends to the body portion 20 and is joined to the end portion on the second tip portion 60 side of the stranded coil 12 by brazing. Also as a material for a principal portion of the second coil 65, stainless steel is used in the case of the present embodiment. Meanwhile, only a portion with a length on the order of 30 mm at the tip side portion of the second coil 65 is a marker part 65a formed of radiopaque metal such as platinum.

The shape of the core wire 14 constituting the first tip portion 30 and the second tip portion 60, namely a combination of the plurality of taper parts and the small diameter part and the dimensions thereof, are not restricted to those described above. The above-described shape of the core wire 14 may take a variety of forms so long as satisfying the later-described conditions for flexural stiffness and tip loads.

The graphs in Figs. 5 and 6 show results of measurement of flexural stiffness of the guidewire 10 illustrated in Figs. 1 to 4, after the production thereof, at predetermined distances from the tip with use of a measurement device 80 illustrated in Fig. 7.

The measurement device 80 has a configuration where two supports 81a and 81b are mounted on a stage 83 at a spacing of a distance D, and thereabove, a load cell 82 having a contact 82a is disposed. In this measurement, the guidewire 10 is mounted on the supports 81a and 81b. Subsequently, the contact 82a of the load cell 82 descends toward the center between the supports 81a and 81b, to press a side portion of the guidewire 10. By this pressing, a load at the time of making the guidewire 10 descend by a predetermined distance is measured.

The first tip portion 30 and the second tip portion 60 are flexible, fine portions as an object of this measurement. It is therefore necessary to measure the flexural stiffness of these parts highly accurately. Hence in this measurement, the distance D between the supports 81a and 81b is set to 5.0 mm, and the contact 82a is descended at 2.5 mm/sec. A load at the time of pressing the guidewire 10 downward by 1.0 mm from the side is then measured.

In the measurement of the flexural stiffness of the body portion 20, the accuracy to a degree for the cases of the first tip portion 30 and the second tip portion 60 is not required. Therefore in this measurement, the distance D between the supports 81a and 81b is set to 20.0 mm, the contact 82a is descended at 5.0 mm/min, and a load at the time of pressing the guidewire 10 downward by 10.0 mm from the side is then measured.

The graph in Fig. 5 shows results of the measurement performed sequentially from the respective tips of the first and second tip portions 30 and 60 toward the body portion 20. Further, Fig. 6 shows the measurement results of the tip portions in an enlarged manner.

In Figs. 5 and 6, the graph indicated by black triangles show the flexural stiffness [mN] of the first tip portion 30. Meanwhile, the graph indicated by white squares shows the flexural stiffness [mN] of the second tip portion 60.

Concerning the graph of the first tip portion 30, the portions indicated by R1, R2, and R3 correspond to the first taper part 31, the first small diameter part 32, and the second taper part 33, respectively. The portion indicated by RM and a further portion show the body portion 20 connected to the proximal end portion of the first tip portion 30.

Similarly, concerning the graph of the second tip portion 60, the portions indicated by A1 and A2 correspond to the third taper part 61 and the fourth taper part 62, respectively. The portion indicated by AM and a further portion show the body portion 20 connected to the proximal end portion of the second tip portion 60.

As illustrated in Fig. 5, the flexural stiffnesses of the first tip portion 30 and the second tip portion 60 are both set so as to increase in a continuous or stepwise manner from the tips toward the body portion 20. Herein, considering inside ranges of 120 mm from each of the tips which are indicated by both distance ranges L1 and L2, i.e., distance ranges where the first tip portion 30 and the second tip portion 60 are both present, the flexural stiffness of the first tip portion 30 is set high as compared with the flexural stiffness of the second tip portion 60 in a first distance range indicated by L1 on the order of 23 mm from each of the tips. The flexural stiffness of the first tip portion 30 then becomes equal to the flexural stiffness of the second tip portion 60 at the endpoint of the first distance range L1. In the second distance range L2 thereafter, the flexural stiffness of the first tip portion 30 is set low as compared with the flexural stiffness of the second tip portion 60. In other words, in the first distance range L1 and the second distance range L2, the flexural stiffness of the first tip portion 30 moderately increases, whereas the flexural stiffness of the second tip portion 60 relatively sharply increases from a low state.

In a third distance range L3 after L2, with the second tip portion 60 being no longer present, the measurement range has shifted to the body portion 20. On the other hand, the flexural stiffness of the first tip portion 30 is still held in a low state. The measurement range shifts to the body portion 20 from the endpoint of the third distance range L3.

The flexural stiffness of the body portion 20 is fixed. Therefore, after the shift of the measurement ranges from both the first tip portion 30 and the second tip portion 60 to the body portion 20, the measurement results of the flexural stiffness of the first tip portion 30 and the second tip portion 60 become equal to one another.

The reason that such flexural stiffness is set is that the first tip portion 30 is used for the retrograde approach, so the first tip portion 30 needs to pass through a stenosis, bent or tortuous blood vessel called a collateral channel. For this reason, the first tip portion 30 is basically required to be long and flexible over the distance ranges L1, L2, and L3. Meanwhile, at the time of the first tip portion 30 being exposed to the outside of the body by the retrograde approach, a medical catheter such as a balloon catheter is inserted from the tip of the first tip portion 30. Facilitating this insertion is thus required. Therefore, the tip portion of the first tip portion 30 preferably has a later-described rigid tip load. Accordingly, there is a need to make the stiffness of the first tip portion 30, indicated by the distance range L1, higher than the stiffness of the second tip portion 60 in the corresponding portion.

As described above, the axial length of the first tip portion 30 is set not smaller than 150 mm, and is about 280 mm in the present embodiment. This length is set such that the first tip portion 30 is provided with a portion flexible enough to flexibly follow a bent or tortuous blood vessel at the time of approaching a target treatment site via the collateral channel. When this length is smaller than 150 mm, a portion to enter into the collateral channel is excessively short, and the length is thus not sufficient for passage through a plurality of tortuosities of the collateral channel which are sharply bent.

On the other hand, the second tip portion 60 is used for the antegrade approach after the retrograde approach. More specifically, the second tip portion 60 is used for a blood vessel having no stenosis, a stenosis already passed through by the retrograde approach, or a stenosis in a state not as severe as the stenosis as the object of the retrograde approach. Hence the flexible part as long as that of the first tip portion 30 need not be used. However, since the second tip portion 60 is used for a variety of applications inside the blood vessel in a relatively nonsevere state as described above and needs to prevent damaging of the blood vessel as much as possible, the tip portion of the second tip portion 60 is required to have flexibility.

Further, when used for the antegrade approach, the second tip portion 60 needs to support the medical catheter such as the balloon catheter. This requires a portion, closer to the body portion 20 side than the range on the order of 20 to 30 mm from the tip, to have a certain amount of stiffness. This is because this part plays an important role for receiving reactive force from the medical catheter at the time of treatment. It is thus needed to increase the stiffness of the second tip portion 60 within the range on the order of 20 to 30 mm from the tip. Hence, within this range, values of the stiffness of the first tip portion 30 and the second tip portion 60 are reversed. In the present embodiment, therefore, the endpoint of the first distance range L1 where the stiffness of the first tip portion 30 and the stiffness of the second tip portion 60 are reversed is set at a position on the order of 23 mm.

To show such an increase in stiffness, the axial length of the second tip portion 60 is set in the range on the order of 40 to 150 mm, and is about 120 mm in the present embodiment, as described above. In the case of an axial length smaller than 40 mm, the support-requiring portion which is closer to the body portion 20 side than the range on the order of 20 to 30 mm from the tip in the second tip portion 60 is excessively short. This leads to an excessively sharp change in stiffness. In the case of the axial length of the second tip portion 60 exceeding 150 mm, the support-requiring portion which is closer to the body portion 20 side than the range on the order of 20 to 30 mm from the tip in the second tip portion 60 is excessively long, and thereby the change in stiffness in this portion is excessively mild. This causes deterioration in operability.

The flexural stiffness of the body portion 20 also needs to be set low as compared with a normal guidewire. This is because, as the body portion 20 also passes through the inside of the heart including the collateral channel, excessively high stiffness of the body portion 20 might cause load on the heart. The flexural stiffness of the body portion 20 is preferably from 3000 to 6000 [mN], and is adjusted to about 4300 [mN] in the present embodiment. The flexural stiffness of the body portion 20 tends to become larger with increase in outer diameter of the body portion 20. However, as in the present embodiment, combining the core wire 14 with the stranded coil 12 allows the flexural stiffness of the body portion 20 to be set low while a desired diameter is held in the body portion 20.

The graph in Fig. 8 shows results of measuring tip loads [mN] of the first tip portion 30 and the second tip portion 60 after production of the guidewire 10 illustrated in Figs. 1 to 4. A measurement device 90 is illustrated in Fig. 9 and has a configuration where a measurement part 93a is disposed on a weight measurement device 93, and thereabove, a cylindrical supporting part 92 for supporting the guidewire 10 is disposed. The distance h between the lower end of the supporting part 92 and the measurement part 93a is set to 30 mm. With this configuration, the guidewire 10 is inserted through the inside of the cylindrical supporting part 92, and is substantially vertically mounted on the measurement part 93a on the weight measurement device 93 such that the tip of the guidewire 10 comes into contact therewith. With such a configuration, the respective tips of the first tip portion 30 and the second tip portion 60 sequentially press the measurement part 93a. The maximum load at the time of pressing is then measured.

In Fig. 8, the left-hand bar graph indicates the tip load [mN] of the first tip portion 30, while a righthand bar graph indicates the tip load [mN] of the second tip portion 60.

In these graphs, the tip load [mN] of the first tip portion 30 is about 27.0 [mN], and the tip load [mN] of the second tip portion 60 is about 12.0 [mN].

As thus described, the first tip portion 30 is used for the retrograde approach. Therefore, the first tip portion 30 basically needs to have a long, flexible tip. However, since a medical catheter such as a balloon catheter is inserted from the tip side, the tip load of the first tip portion 30 is set high for facilitating the insertion. It was proved from results of experiments conducted by the present inventors and others that the tip load which allows relatively easy insertion of medical catheters is on the order of 24 to 45 [mN]. When the tip load is below 24 [mN], the tip is so soft as to make insertion of medical catheters difficult. On the other hand, when the tip load exceeds 45 [mN], the tip is so hard as to cause a risk such as damaging of a blood vessel, and is thus unsuitable for actual use. In the case of the present example, the stiffness of the first taper part 31, the first small diameter part 32, the second taper part 33, and the like in the core wire 14 are adjusted so as to make the tip load of the first tip portion 30 be about 27.0 [mN] which was described above.

On the other hand, the second tip portion 60 is used for the antegrade approach after the retrograde approach. That is, the second tip portion 60 is used for a blood vessel having no stenosis, a stenosis already passed through by the retrograde approach, or a stenosis in a state not as severe as the stenosis as the object of the retrograde approach. In order to operate the second tip portion 60 inside a blood vessel in a relatively nonsevere state and prevent as much as possible the tip portion from damaging the blood vessel, the tip load is set relatively low. It has been found from the cases treated by guidewires used for general antegrade approaches that the tip load of such a flexible guidewire is preferably the order of 8 to 20 [mN]. For this reason, the stiffnesses of the third taper part 61, the fourth taper part 62, and the like in the core wire 14 are adjusted such that the tip load of the second tip portion 60 is in the above range (about 12.0 [mN] described above in the case of the present example).

Based upon the above configuration, there is described a function of the guidewire 10 of the present embodiment in the case of using the guidewire 10 for a cardiac operation.

Figs. 10 to 14 schematically illustrate an aorta 310 of a heart, a left coronary artery 320, a right coronary artery 330, and the like inside the body 300 of a patient.

For example, when a stenosis 350 is present in the right coronary artery 330, the antegrade approach is a method for the guidewire being inserted through from the direction of the arrow a1, i.e., a direction of the right coronary artery 330, to approach the stenosis 350. On the other hand, the retrograde approach of the present embodiment is a method for the guidewire being inserted through sequentially from the direction of the left coronary artery 320 toward the directions of the arrows r1, r2, and r3, to approach the stenosis 350 via a collateral channel 340.

In operation steps prior to the use of the guidewire 10, guiding catheters 100 and 200 are disposed in the left coronary artery 320 and the right coronary artery 330, respectively. The tip of the guiding catheter 100 is engaged with a left coronary artery ostium 320a of the left coronary artery 320. Meanwhile, the tip of the guiding catheter 200 is engaged with a right coronary artery ostium 330a of the right coronary artery 330.

Further, before the use of the guidewire 10 of the present embodiment, an operation is normally performed where a guidewire generally used for the retrograde approach (hereinafter referred to as an initially used guidewire) is made to reach the stenosis 350 by the retrograde approach and then pass through the stenosis 350, and this initially used guidewire is thereby made to reach the right coronary artery ostium 330a of the right coronary artery 330. Simultaneously with this, a thin-diameter catheter 400 called a microcatheter is inserted through by the retrograde approach along the initially used guidewire. Examples of such a catheter 400 include a catheter disclosed in JP 2008-264696A of the present applicant.

From this state, the initially used guidewire is removed to the outside of the body. Thereby, the left coronary artery ostium 320a of the left coronary artery 320 and the right coronary artery ostium 330a of the right coronary artery 330 come into the state of being connected with each other by a lumen of the microcatheter 400.

It is to be noted that, only portions of the guiding catheters 100 and 200 and the microcatheter 400 are illustrated for the sake of understandability of the figures. Further, the guidewire 10 is indicated by a solid line, the first tip portion 30 side thereof is indicated by a black arrow, and the second tip portion 60 side thereof is indicated by a white arrow. The microcatheter 400 is indicated by a broken line.

From this state, a treatment for expanding the stenosis 350 with a balloon catheter 450 is performed by use of the guidewire 10. First, as illustrated in Fig. 10, the first tip portion 30 of the guidewire 10 is made to proceed sequentially toward the directions of the arrows r1, r2, and r3 while passing through the inside of the microcatheter 400. After the further procession, the first tip portion 30 enters into the right guiding catheter 200, passes through this right guiding catheter 200, and is exposed to the outside of the body (Fig. 11). As this guidewire 10 has a sufficient length, the second tip portion 60 is still exposed to the outside of the body in this state.

In such a manner, the guidewire 10 proceeds from a first coronary artery side to a second coronary artery side. The vessel diameter of the collateral channel 340 through which the guidewire 10 passes at this time is small, and in the vicinities of this collateral channel 340, a plurality of bifurcations 311a, 311b, and 311c having relatively acute angles are present (see Fig. 10). Even when the guidewire 10 passes through such a collateral channel 340 and proceeds from a main vessel 321 of the left coronary artery 320 to a main vessel 331 of the right coronary artery 330, since the first tip portion 30 thereof has a long and flexible tip portion, the guidewire 10 can proceed while following the plurality of bifurcations 311a, 311b, and 311c. Further, the flexural stiffness of the body portion 20 is set low as described above. For this reason, even in the case of disposing the guidewire 10 inside the body such that the guidewire 10 enters into the body from a first coronary artery side and gets out thereof from a second coronary artery side, thereby to reciprocate with the inside of the heart in the vicinity of the collateral channel 340 taken as a turn-round, acting of an excessive load on the heart or the blood vessel is prevented as much as possible.

When the first tip portion 30 is exposed from the right guiding catheter 200 side to the outside of the body, the microcatheter 400 is pulled back to the vicinity of the exit of the collateral channel 340 (Fig. 12).

Further, the guidewire 10 is pulled by the first tip portion 30 side to the outside of the body. Further, with this pulling, the second tip portion 60 enters into the body. Specifically, the second tip portion 60 moves sequentially along the directions of the arrows r1 and r2, to reach a branching vessel 332 branched from the main vessel 331 of the right coronary artery 330.

Thereafter, the operator operates the first tip portion 30 side so as to cause the second tip portion 60 to move to an appropriate place such as the branching vessel 332 having no stenosis and the like, for example as illustrated by 60a in Fig. 12. In other words, the second tip portion 60 is positioned in an appropriate place by means of the antegrade approach.

Further, depending upon the conditions, the position of the second tip portion 60 may be moved to and positioned in a peripheral-side vessel 332a of the branching vessel 332 by the antegrade approach, as illustrated by 60b in Fig. 12.

In this manner, the second tip portion 60 may be moved by the antegrade approach so that the guidewire 10 is allowed to be disposed in an appropriate position. In so doing, the second tip portion 60 is short as compared with the first tip portion 30, but its tip load is set relatively low. Operation of the second tip portion 60 is thereby easily performed, and damaging of the inside of the blood vessel is prevented as well.

In this manner, after disposition of the second tip portion 60 in a safe blood vessel portion, the balloon catheter 450 is inserted from the tip side of the first tip portion 30. Herein, the tip load of the first tip portion 30 is set to have hardness with which a medical catheter such as the balloon catheter 450 is easily inserted. This can facilitate insertion of the balloon catheter 450 into the first tip portion 30.

The inserted balloon catheter 450 passes through the inside of the right guiding catheter 200 along the guidewire 10 and enters into the main vessel 331 of the right coronary artery 330, to reach the stenosis 350.

In this state, the balloon catheter 450 is expanded, so that a treatment to dilate the stenosis 350 is performed.

Further, there are cases where a stenosis 360 is present in a peripheral-side vessel part 331a of the main vessel 331 in the right coronary artery 330. In this case, after completion of treatment of the stenosis 350 as the principal object, the first tip portion 30 side which is outside the body is operated to cause the second tip portion 60 to move to the peripheral-side vessel part 331a of the main vessel 331. Specifically, the second tip portion 60 is moved to the vessel part 331a by means of the antegrade approach.

Then, as illustrated in Fig. 13, after allowing the second tip portion 60 to pass through the stenosis 360, the balloon catheter 450 is moved to the stenosis 360 along the guidewire 10, so that a treatment to dilate the stenosis 360 is performed.

Normally, the stenosis 350 as the principal object is a stenosis in the most severe state, and it thus becomes an object part of the retrograde approach. The stenosis 360 treated thereafter is a portion in a state not as severe as the stenosis 350, and thus is suitably treated by making the second tip portion 60 enter thereinto by means of the antegrade approach.

In such a manner as above, upon completion of the treatments of the stenoses 350 and 360, the balloon catheter 450 and the guidewire 10 are pulled out from the right guiding catheter 200 side, i.e., the first tip portion 30 side. Subsequently, the microcatheter 400 is also pulled out from the left guiding catheter 100 side, to complete the treatment.

As thus described, the guidewire 10 of the present embodiment is capable of continuously using the first tip portion 30 used for the retrograde approach and the second tip portion 60 used for the antegrade approach. Hence a stenosis in a severe state becomes treatable, which has been difficult by the conventional operation. Further, a stenosis other than the stenosis treated by the retrograde approach may also be successively treated by the antegrade approach.

In the first embodiment described above, the stiffness of the body portion 20 is adjusted through use of the stranded coil 12 constituted by the core wire 14 and the plurality of strands. In this case, forming a desired outer diameter from the stranded coil 12 and using the core wire 14 that fits therewith enables subtle adjustment of the outer diameter and the stiffness of the guidewire 10. Further, the stranded coil 12 generally has a characteristic of having high torque transmissibility. For this reason, using the stranded coil 12 enables achievement of a flexible guidewire with high torque transmissibility.

Moreover, surrounding the first tip portion 30 and the second tip portion 60 by the first coil 35 and the second coil 65 with substantially the same diameter as the stranded coil 12 enables achievement of such a shape that the guidewire 10 has substantially the same outer diameter as a whole. As the coils have substantially the same diameter, there is an advantage that, even when the guidewire 10 is inserted into the body or pulled toward the outside of the body, or when an operation such as rotation is performed inside the body, the guidewire 10 getting stuck inside the catheter and inside the body is prevented as much as possible.

However, the configuration as in the foregoing first embodiment is not indispensable. The guidewire of the embodiments of the present invention may have a configuration as in a second embodiment illustrated in Fig. 14. In a body portion 520 of a guidewire 510 illustrated in Fig. 14, the outer diameter and stiffness of the body portion 520 of the guidewire 510 are adjusted only by a core wire 514 without using a stranded coil. Also in this case, a first tip portion 530 and a second tip portion 560 are surrounded by a first coil 535 and a second coil 565 which are constituted by single strands from the viewpoint of safety.

With this configuration, the guidewire 510 has a simple configuration as a whole.

Further, as a guidewire 610 of a third embodiment illustrated in Fig. 15, the guidewire may have a configuration where the core wire is surrounded by the same stranded coil in the first tip portion, the second tip portion, and the body portion. A first tip portion 630 of the guidewire 610 illustrated in Fig. 15 is surrounded by a stranded coil 612 constituted by a plurality of strands, as is a body portion 620. As for stiffness of the first tip portion 630, the stiffness may be changed only by decreasing the diameter of a core wire 614. However, in the case of the present embodiment, in addition to decreasing the diameter of the core wire 614 in a tapering manner, sequentially decreasing the number of strands constituting the stranded coil 612 as illustrated by 630a, 630b, and 630c increases the flexibility of the stranded coil 612 toward the tip. Thereby, the stiffness of the first tip portion 630 is adjusted.

Similarly, the stiffness of the first tip portion 630 may also be adjusted only by decreasing the number of strands constituting the stranded coil 612 while the core wire 614 is previously formed to have a cylindrical shape with a fixed small diameter.

Further, the stiffness of the stranded coil 612 may also be adjusted by performing centerless grinding or electrolytic polishing on the outer surface of the stranded coil 612 to decrease the diameter of the strands.

In the embodiments described above, the retrograde approach has been described where the guidewire is made to enter inside from a first coronary artery and be exposed to the outside from a second coronary artery. However, the guidewire of the embodiments of the present invention may also be used for a retrograde approach where a guidewire is made to be exposed to the outside from the same coronary artery as a coronary artery from which the guidewire entered inside.

Further, although the case of the operation for making a continuous shift from the retrograde approach to the antegrade approach is exemplified in the embodiments described above, the first tip portion and the second tip portion may also be separately used for the retrograde approach or the antegrade approach.

Moreover, in the embodiments described above, the core wire at the respective tip portions of the first tip portion and the second tip portion is formed in a taper shape having a circular cross section. However, this core wire may be formed into a variety of tip shapes, such as a taper shape having an inclined plane and a shape with a plurality of flat parts having a substantially rectangular cross section and a thickness reducing toward the tips for shaping of the tip portion and the like.

While the invention has been shown and described in detail, the foregoing description is in all aspects illustrative and not restrictive. It is therefore understood that numerous modifications and variations can be devised without departing from the spirit and scope of the invention.

## Claims

1. A guidewire comprising:
a body portion; and
a first tip portion and a second tip portion that are provided at ends of the body portion, wherein
flexural stiffnesses of the first tip portion and the second tip portion increase in a continuous or stepwise manner from the respective tips of the first tip portion and the second tip portion toward the body portion,
the flexural stiffness of the first tip portion is not smaller in value than the flexural stiffness of the second tip portion in first distance ranges from the respective tips of the first tip portion and the second tip portion toward the body portion, and
the flexural stiffness of the first tip portion is smaller in value than the flexural stiffness of the second tip portion in second distance ranges beyond the respective first distance ranges of the first tip portion and the second tip portion toward the body portion.

2. The guidewire according to claim 1, wherein the first tip portion is larger in tip load than the second tip portion.

3. The guidewire according to claim 2, wherein the tip load of the first tip portion is not smaller than 24 mN.

4. The guidewire according to any of claims 1 to 3, wherein the first tip portion is larger in axial length than the second tip portion.

5. The guidewire according to claim 4, wherein the axial length of the first tip portion is not shorter than 150 mm.

6. The guidewire according to any of claims 1 to 5, wherein the guidewire has such an overall length as to allow the first tip portion to be inserted into a body, pass through an inside of a heart, be turned back and exposed to an outside of the body in a state where the second tip portion is exposed to the outside of the body.

7. The guidewire according to any of claims 1 to 6, wherein the body portion is configured with a core wire being inserted through an inside of a stranded coil constituted by a plurality of strands.
